# EUROPEAN PATENT APPLICATION

(11) **EP 1 564 304 A1**
(43) Date of publication of application: **17.08.2005**
(21) Application number: 04029074.4
(22) Date of filing: 08.12.2004
(51) Int. Cl.: C12Q 1/68

(54) **Method of DNA array construction, gene expression analysis, and discovery of useful genes**

(30) Priority: 10.02.2004 JP 2004032813
(71) Applicant: Hitachi Ltd., Tokyo (JP)
(72) Inventor: Yokoi, Takahide, 1-6-1 Marunouchi Chiyodaku Tokyo100-8220 (JP); Minowa, Takashi, 1-6-1 Marunouchi Chiyodaku Tokyo100-8220 (JP); Saito, Toshiro, 1-6-1 Marunouchi Chiyodaku Tokyo100-8220 (JP); Kaku, Yoshiko, 1-6-1 Marunouchi Chiyodaku Tokyo100-8220 (JP)
(74) Representative: Strehl Schübel-Hopf & Partner

(57) **Abstract**

Methods for global monitoring of gene expression and searching for useful genes that are targeted to organisms lacking sequence information are realized by providing low-cost and efficient DNA arrays. A genomic library constructed from randomly cleaved genomic DNA fragments is directly fixed on a substrate that allows the library to be individually recognized . In this way, global monitoring of gene expression can be carried out without being limited by the amount of gene sequence information. Further, plasmids that are detected by a constructed random genomic DNA array are fragmented into shorter DNAs, which are fixed on another substrate to construct a sub DNA array. With the use of this sub DNA array, gene expression is analyzed and useful genes are searched.

## Description

### FIELD OF THE INVENTION

The present invention relates to a DNA array targeted to living organisms, particularly to microorganisms, a method for global monitoring of gene expression with the use of the array, and a method of searching for useful genes.

### BACKGROUND OF THE INVENTION

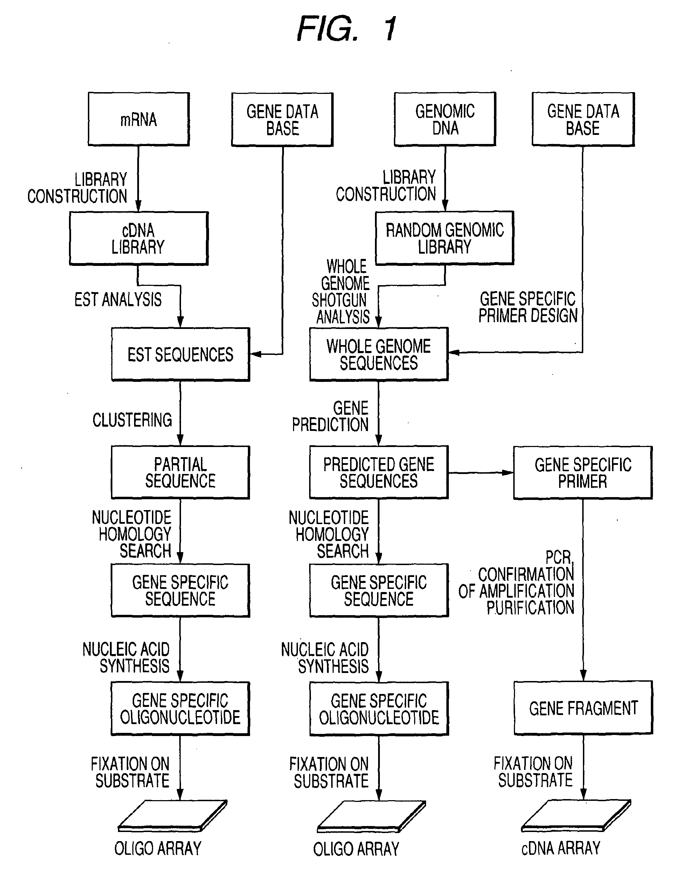

The use of a high density DNA array enables global monitoring of gene expression and is useful for analysis of gene expression pattern in specific phenotype. Generally, the high density array is obtained by fixing on a substrate cDNA sequences or oligonucleotides as probes. An example of construction flow of a conventional DNA array is explained with reference to Fig. 1. For preparation of an oligo array, sequencing of cDNAs synthesized from mRNAs or genomic DNA is carried out. Subsequently, gene prediction and search for specific sequences are performed using the obtained sequences, and oligonucleotides synthesized based on the sequence information are fixed on a substrate as probes. For the construction of cDNA array, primers to specifically amplify individual genes are synthesized based on the predicted gene sequences obtained by the genomic analysis, and each gene is amplified by PCR and confirmed, followed by fixing as probes the amplified and purified DNAs on a substrate.

On the other hand, the use of DNA arrays utilizing a random genomic library has been reported recently (Non-patent document 1, Non-patent document 2). In these reports, DNA fragments to be fixed on a substrate are prepared by PCR using an obtained genomic library, thus allowing to construct a DNA array.

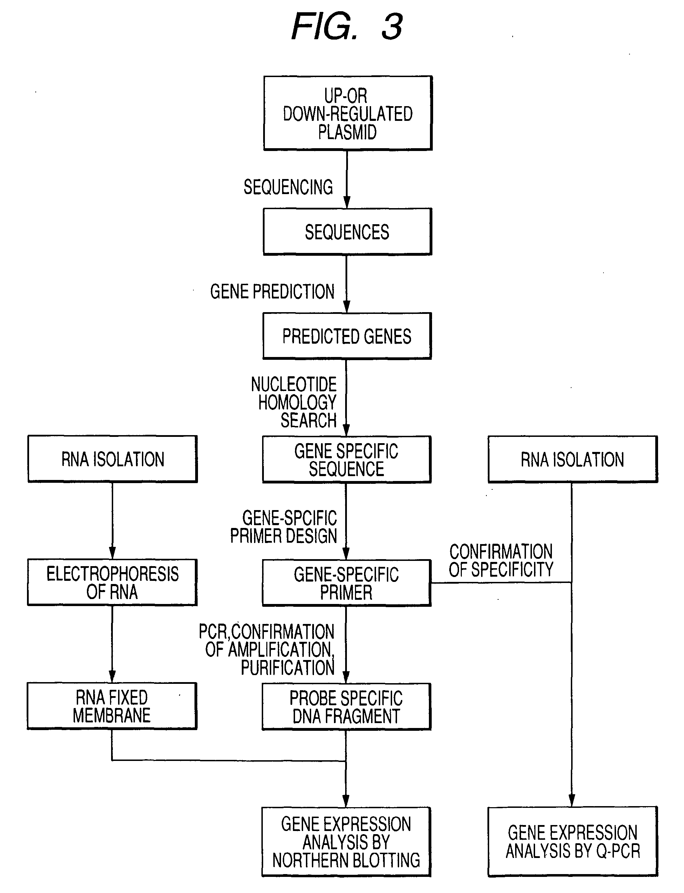

An example of conventional flow of searching for up- and down-regulated genes from a plurality of gene candidates is shown in Fig. 3. Conventionally, a plurality of consecutive analysis procedures were required including 1) sequencing of DNA fragments of which up- or down-regulation was confirmed by a DNA array, 2) gene prediction, and 3) expression analysis of predicted individual genes by Northern blotting or RT-PCR.

[Non-patent document 1] Zaigler et al., Mol. Microbiol. 48:1089-1105, 2003
[Non-patent document 1] Parro et al., Proc. Natl. Acad. Sci. USA 100:7883-7888

To use cDNA sequences as probes, information on individual gene sequences and isolation of DNA fragments having the respective gene sequences are required. For oligonucleotide probes, information on ORF sequences or EST sequences, and more desirably, on genome sequences, is required for their design. Since acquisition of these sequence information is very costly, species of organisms applicable to a high density array are limited.

Eukaryotic mRNAs have poly A sequences in common at their 3' end, and by taking advantage of this feature, cDNA clones corresponding to individual gene sequences can be recovered, and at least part of individual gene sequences can be analyzed by a method generally called EST analysis. However, mRNAs of prokaryotes do not have common sequences such as poly A. Therefore, EST analysis is difficult, and a high density DNA array with cDNA clones as probes such as that used for eukaryotes has not yet been constructed.

Since the high density array has a feature that a great number of genes can be analyzed for their expression, highly global monitoring is desired in many cases. However, conventional high density arrays are at high risk for missing important gene sequences even when constructed on the basis of whole genome sequences because 1) a gene prediction program for detection of ORF regions to be translated into proteins is not perfect and 2) non-coding RNAs that are not translated into proteins are not predictable.

Furthermore, the methods described in Non-patent documents 1 and 2 give rise to unstable PCR results depending on target DNA sequences that should be amplified, and thus there are cases in which amplification is not detected or unnecessary amplification of DNA fragment other than the targeted fragment is observed. Therefore, it is essential to confirm amplification products after PCR. Although sequence information became unnecessary for constructing those DNA arrays, their construction processes still remain complicated.

### SUMMARY OF THE INVENTION

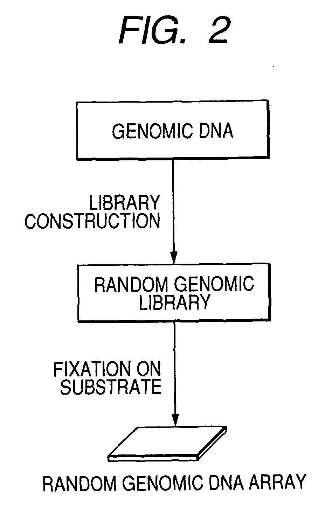

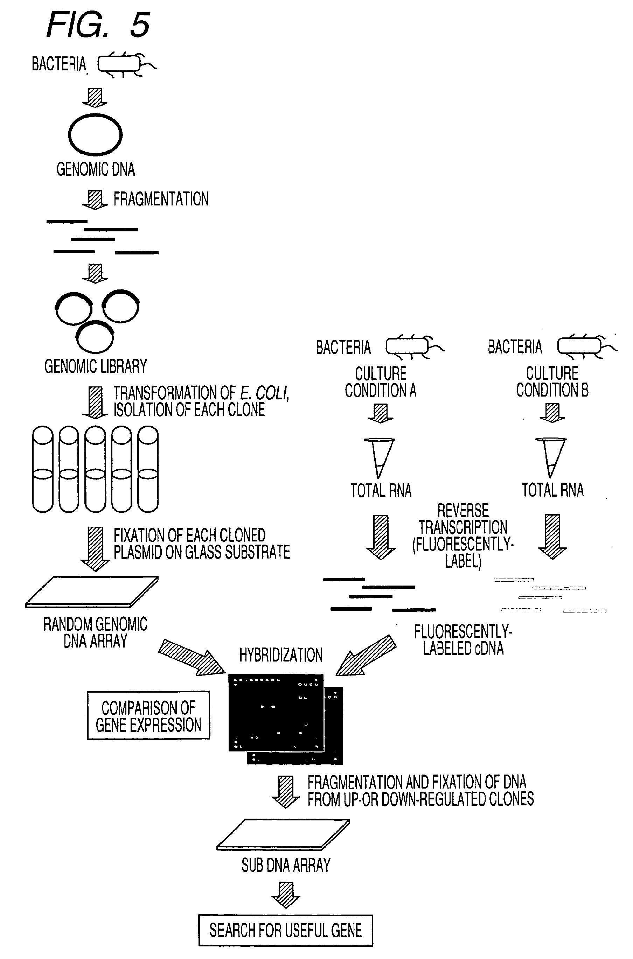

The above problems can be solved by the following construction:
1) As to the array and the method of array construction, a random genomic library is constructed as shown in the upper rows of Fig. 5 that represents a flow of searching for useful genes or as shown in Fig 2 that represents a construction flow of a random genomic DNA array, and each genomic DNA fragment is fixed on a substrate without sequencing. That is, clones isolated from the random genomic library are fixed on the substrate as they are. Since the DNA fragments to be fixed are not prepared by using PCR, it becomes possible to make longer the length of the DNA fragments to be fixed on the substrate and construct a DNA array that is fixed with the DNA fragments of arbitrary length.
   In conventional technology, there is a possibility of missing important genes because gene prediction only from sequences is still imperfect. In the above-mentioned way, not only can this missing be eliminated but also a complicated work to confirm each of a number of up- or down-regulated gene candidates individually is not needed.
2) The method of expression analysis is as is shown in the middle rows of Fig. 5. That is, the random genomic array prepared in 1) described above is supplied with a sample such as fluorescently labeled cDNAs resulted from reverse transcription of RNA, followed by hybridization reaction and expression analysis. For this sample, two or more kinds of RNA including, for example, a control RNA and another RNA obtained by changing culture conditions such as giving certain stimulation may be prepared to carry out their competitive reaction.
3) When the length of the DNA fragments to be fixed on the substrate is made longer, a possibility that a single fixed DNA fragment contains a plurality of gene species is enhanced, thereby making it difficult to identify a gene truly up- or down-regulated. In other words, as shown in Fig. 3, it becomes necessary to carry out a complex gene expression analysis process for individual genes, starting from sequencing DNA fragments containing plural genes.

The details of the present invention are as follows.

The length of genomic DNA fragments to be fixed on the substrate is desirably short and approximately 500 bp that is actually expected to be below one gene or one exon. This is because short genomic DNA fragments are expected to provide more stable PCR results and more accurate expression analysis results. For the purpose of global monitoring of gene expression, choice of shorter lengths of DNA fragments requires, however, preparation of more DNA fragments compared to choice of longer lengths of DNA fragments, and an increase in the number of fragments to be prepared results in an increase in fabrication cost of DNA array. When *E. coli* with a genome size of 4.6 Mbp is used as an example for calculation and when the length of DNA fragments is 500 bp, preparation of 9,200 DNA fragments (4.6 Mbp/500 bp) is required to achieve global monitoring of gene expression with a redundancy of 1 that is minimally needed, while 2,300 DNA fragments (4. 6 Mbp/2, 000 bp) is required in the case where the length of DNA fragment is 2, 000 bp, thus allowing to fabricate a DNA array at lower cost. When a DNA array for fungus species having a larger genome size (ca. 30 Mbp) and exon/intron structure is fabricated at low cost, fixation of DNA fragments with the length of 5 to 10 kbp seems effective. However, efficient and stable amplification of DNA fragments with this range of length as well as unknown sequences becomes more difficult. In other words, the length of DNA fragments to be fixed on a substrate limits the use of PCR for preparation of the DNA fragments, thereby disturbing a decrease in fabrication cost of a DNA array.

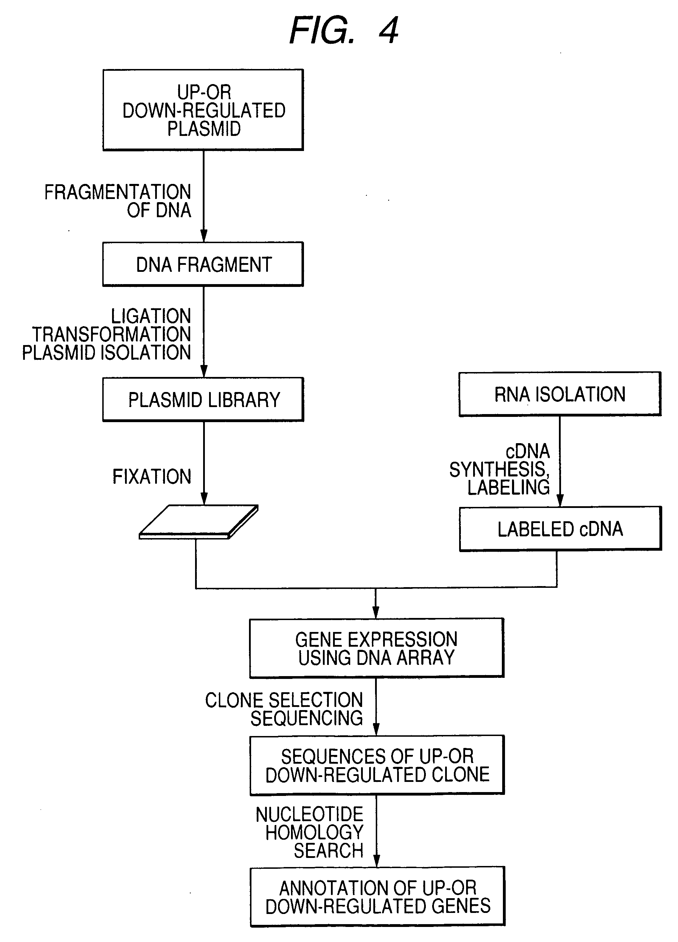

Accordingly, in the present invention, DNAs of up- or down-regulated clones are fragmented and fixed to construct a sub DNA array as shown in the lower part of Fig. 5. The details of this procedure, as shown in Fig. 4, include dividing the DNA fragments, of which up- or down-regulation was confirmed in the constructed random genomic array, into further shorter genomic fragments, specifically ca. 500 bp supposed to be shorter than the length of one gene or one exon, or further shorter fragments, and carrying out expression analysis after construction of another DNA array, thereby allowing global monitoring of genes truly up- or down-regulated. Fixation of shorter fragments makes it possible to identify truly up- or down-regulated genes by sequencing.

It should be noted that analysis targets are narrowed down based on the analysis results from the random genomic DNA array and that further analysis can be performed by fabricating an array with cost reduced. In this case, the genomic DNA fragments selected for analysis targets are fixed on a random genomic DNA array to construct an array with a reduced number of spots of genomic DNA fragments, which is used for repeated analysis. Further, after the genomic DNA fragments selected for analysis targets have been subjected to sequencing, oligonucleotides having their respective sequences may be prepared and fixed on an array. Still further, after the genomic DNA fragments selected for analysis targets based on the analysis results from the random genomic DNA array have been subjected to sequencing, oligonucleotides having their sequences are prepared as primers, and these primers are used for gene expression analysis by quantitative PCR.

In this way, the DNA array for global monitoring of gene expression can be fabricated at low cost and in a simple process regardless of species of organisms or the presence or absence of gene sequence information. Moreover, it is possible to search for useful genes in a global manner by constructing a sub DNA array on the basis of the result obtained from the DNA array.

### BRIEF DESCRIPTION OF THE DRAWINGS

Fig. 1 is an example of construction flow of a conventional DNA array;
Fig. 2 is an example of construction flow of a random genomic DNA array;
Fig. 3 is an example of conventional flow of searching for up- and down-regulated genes from a plurality of gene candidates;
Fig. 4 is an example of flow of searching for up- and down-regulated genes by a sub DNA array;
Fig. 5 is an example of flow of searching for useful genes;
Fig. 6 is an example of the result of plotting signal values obtained with the random genomic DNA array;
Fig. 7 is an example of up- or down-regulation of genes by temperature stimulation, where Fig. 7A shows analysis results from high temperature stimulation, and Fig. 7B shows analysis results from low temperature stimulation;
Fig. 8 is an example of the result of cluster analysis;
Fig. 9 is an example of the result of homology search against *E. coli* K-12 genome data, where Fig. 9A represents the result of homology search of a nucleotide sequence that showed an increased expression at 50 degrees C, and Fig. 9B represents the result of homology search of a nucleotide sequence that showed an increased expression at 16 degrees C; and
Fig. 10 is an outline scheme for construction of sub DNA array.

### DETAILED DESCRIPTION OF THE PREFERRED EMBODIMENTS

The present invention made it possible to carry out global monitoring of gene expression by using DNA fragments derived from a genome as DNA fragments fixed on a substrate, and realized simplification and cost reduction in fabrication of a DNA array by not carrying out sequencing of DNA fragments to be fixed and fixing clones directly.

In addition, the present invention made it possible to select freely the length of DNA fragments, in a range from several kbp to several tens of kbp or longer lengths according to the purpose of expression analysis, by direct fixation of clones. The length of DNA fragments was previously limited to the length that could be amplified by PCR. In order to carry out global monitoring of gene expression, DNA fragments from all gene species are desired to be fixed on the same substrate, while kinds of molecules to be fixed are determined by the genome size of an organism targeted for analysis, the length of DNA fragments to be fixed, the number of DNA fragments fixable on the same substrate, and the cost of substrate fabrication. When the genome size is large, when the number of DNA fragments fixable on the substrate is small, or when the fabrication cost is wanted to be reduced, it is effective to make the length of DNA fragments to be fixed longer.

Since the substrate fixed with a number of DNA fragments for a DNA array can be multi-replicated, two or more monitoring results of gene expression can be compared to each other. In an ordinary DNA array that is fixed with DNA fragments derived from individual gene sequences, the results of comparison of expression analysis are recognized as up- or down-regulation of individual gene species. On the other hand, in the random genomic DNA array of the present invention, detected up- or down-regulation represents the total sum of the changes in fixed gene species because fixed molecules contain a plurality of gene species. The change detected in the random genomic DNA array is derived from the change of mRNA expressed in the living organism targeted for analysis, and therefore an analysis of the condition of the targeted organism can be performed without depending on identification of gene species. An analysis standing on such a viewpoint is particularly useful for process control at a production site of fermentation products using microorganisms. Moreover, this analysis is capable of targeting to even a sequence region that can not be predicted by a general analysis program for prediction of exon region and open reading frame (ORF), for example, a non-coding region that is a sequence region not encoding amino acid sequences.

Based on the result from an analysis performed in advance with the use of the random genomic DNA array of the present invention, molecules to be fixed on an array are selected, thereby allowing a second random genomic array with more specific analysis targets to be constructed. In other words, the present random genomic DNA array can be utilized not only for analysis itself but also for screening of analysis targets.

Next in the present invention, DNA fragments of which up- or down-regulation was confirmed in the DNA array constructed beforehand are divided into further shorter genomic fragments, specifically into fragments of approximately 500 bp supposed to be shorter than the length of one gene or one exon, and additional DNA array (sub DNA array) is constructed and used for carrying out expression analysis, thereby allowing global monitoring of genes truly up- or down-regulated.

As described above, the DNA array constructed according to the present invention is fixed with DNA fragments containing a plurality of gene species, and therefore up- or down-regulation to be detected represents the total sum of the changes in fixed gene species. The analysis of up- or down-regulation of individual genes in DNA fragments containing a plurality of genes often requires a series of multiple analyses such as 1) sequencing of DNA fragments of which up- or down-regulation was confirmed by the DNA array, 2) gene prediction, and 3) expression analysis of each of the predicted genes by Northern blotting or RT-PCR (Fig. 3).

It is complicated in some cases to carry out these analyses on a plurality of DNA fragments confirmed by global monitoring of gene expression using the DNA array constructed by the present invention.

Hence, in the present invention, a sub DNA array in which the DNA fragments up- or down-regulated are divided into shorter fragments and fixed on a substrate is constructed, and genes up- or down-regulated are further subjected to identification (Fig. 4). The length of the DNA fragments to be fixed is made to approximately 500 bp or shorter than this, and thus it is supposed that the kind of gene or exon contained in a fixed DNA fragment is one or less. For this reason, the changes in expression analyzed by the use of the present sub DNA array are expected to reflect accurate expression changes of individual genes truly up- or down-regulated. Moreover, since the preparation of the DNA fragments fixed on the sub DNA array does not include a step often resulting in inaccuracy such as gene prediction, this method is considered to be the one that allows global search for expressed genes not only in prokaryotes but also in eukaryotes that are difficult in gene prediction.

An outline of the whole present invention is summarized in Fig. 5. The expression analysis by the use of the random genomic DNA array having a genomic library fixed directly on a substrate, and the search for genes by the use of only the sub DNA array can be carried out independently. However, the combination of these makes it possible to achieve the global monitoring of gene expression and the search for genes in a short time and at low cost.

In the present invention, a slide glass treated with poly-lysine is used for the substrate on which genomic DNA fragments are bound, and the DNA fragments are fixed by a spotting method. However, the substrate is not particularly limited to this as long as genomic DNA fragments can be fixed, and the fixing method is not limited as well.

For cDNA probes that are hybridized to the DNA array or sub DNA array, a variety of cDNA probes can be used depending on the purposes. These include, for example, isotope-labeled probes or chemically labeled probes of cDNAs derived from mRNAs obtained under various culture conditions, cDNAs derived from a mutant strain of the organism used for preparation of the random genomic library, or cDNAs derived from a relative strain of the organism used for preparation of the random genomic library.

Gene expression can be quantified by hybridizing these probes on the DNA array or sub DNA array and then measuring the annealed probes with measurement equipment using the label bound to the probes as an index. Changes in gene expression can be directly detected by labeling two kinds of cDNAs to be used for comparison with two kinds of fluorescent substances such as Cy3 and Cy5, respectively, followed by subjecting to competitive hybridization on the same substrate at the same time. Alternatively, it is possible to compare one result with another result from expression analysis on an additional piece of the substrate.

It should be noted that general procedures necessary for gene analysis such as digestion and ligation of DNA, transformation of *E. coli,* gene sequencing, and labeling of cDNA can be carried out according to commonly used procedures.

### EXAMPLES

The present invention is explained in more detail by means of the following examples, but is not limited only to these examples.

### Example 1 Construction of random genomic DNA array

Genomic DNA was isolated from *E. coli* JM-109 by a CTAB method. The isolated genomic DNA was subjected to partial digestion with restriction enzyme Taq αI, and a group of digested DNA fragments of ca. 2 kbp were recovered from agarose gel.

A vector (pUC19) for linking to the digested DNA fragments was cleaved with restriction enzyme *ACC* I, and further its end was dephosphorylated with modification enzyme CIAP.

The enzyme-treated pUC19 was mixed with the digested genomic DNA fragments that had been recovered from *E. coli* and ligated to them with a ligase, followed by introduction into competent *E. coli* XLI-Blue MRF' cells.

Plasmids carrying the genomic DNA fragments were recovered from arbitrary 3,000 colonies of *E. coli,* transformed with plasmids and grown on an agar medium, by an alkaline-SDS method, yielding a random genomic library as 3,000 clones of plasmids. This plasmid library contains digested genomic fragments of ca. 2 kbp respectively. Therefore, the 3,000 clones represent genomic fragments of 6 Mbp (2 kbp × 3,000 clones), and are assumed to contain *E. coli* genome having a genome size of 4.2 Mbp with a redundancy of approximately 1.4.

The recovered 3,000 clones of plasmids were spotted on a slide glass using Spotter SP-Bio (manufactured by Hitachi Software Engineering Co., Ltd.) and fixed by a heat treatment at 80 degrees C.

For the purpose of evaluating the accuracy of gene expression analysis by the random genomic DNA array, the total RNA was isolated from *E. coli* JM-109 cells grown in liquid LB media at 37 degrees C up to an optical density of 1.0 at 600 nm.

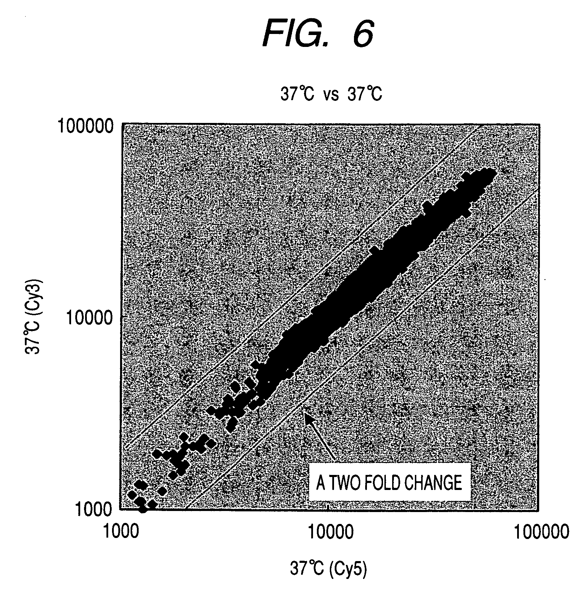

The isolated total RNA was converted to cDNAs with a reverse transcriptase using random 6-mer oligonucleotides as primers. The synthesized cDNAs were divided into two portions, and these portions were labeled with Cy3 and Cy5, respectively, followed by subjecting to competitive hybridization on the same random genomic DNA array. The result of plotting obtained signal values is shown in Fig. 6. Signals from the spots fixed on the array are distributed within at most two fold up- or down-regulation, and this result suggests that clones with at least two fold up- or down-regulation observed in a comparative experiment of gene expression are considered different in expression.

Next, for the purpose of analyzing up- or down-regulation of gene induced by temperature stimulation, the *E. coli* cells were cultured under three different temperature conditions. The culture conditions used were as follows: *E. coli* JM-109 cells grown in the liquid LB media at 37 degrees C up to an optical density of 1.0 at 600 nm were further allowed to grow 1) continuously at 37 degrees C, 2) at 50 degrees C for 7 min in the LB media, and 3) at 16 degrees C for 60 min in the LB media. The total RNA was isolated from each of the cultured *E. coli* cells.

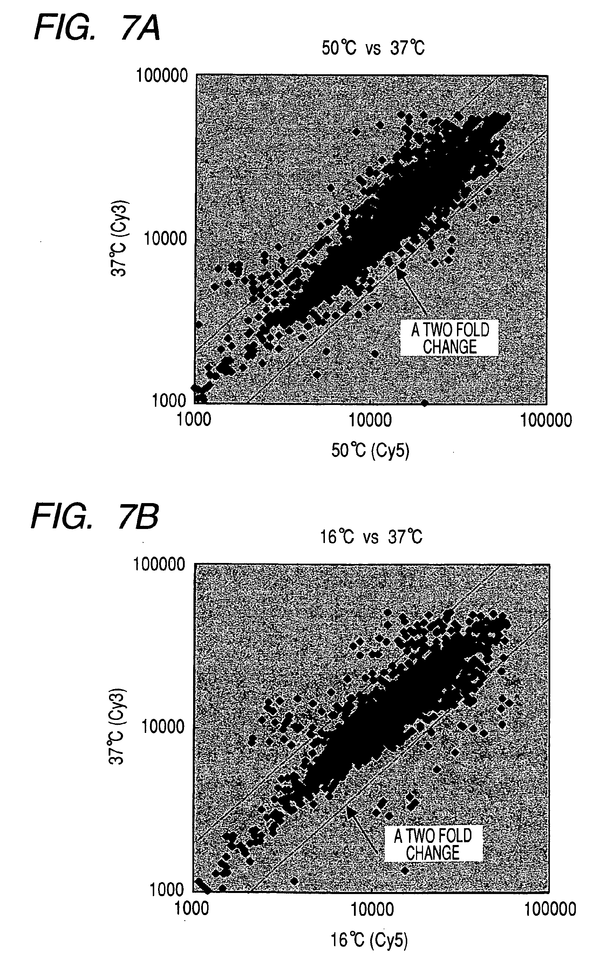

After the three kinds of the total RNA were converted to cDNAs with a reverse transcriptase using random 6-mer oligonucleotides as primers, the cDNAs derived from *E. coli* cells cultured at 37 degrees C were labeled with Cy3, and the cDNAs derived from those cultured at 50 degrees C were labeled with Cy5 for analyzing up- or down-regulation of gene caused by the high temperature stimulation, followed by competitive hybridization on the same random genomic DNA array. Fig. 7A shows the analysis results from the high temperature stimulation, where the horizontal axis represents signal values derived from the culture at 50 degrees C and the vertical axis represents signal values derived from the culture at 37 degrees C in the plot.

On the other hand, for the analysis of up- or down-regulation of gene caused by the low temperature stimulation, the cDNAs derived from *E. coli* cells cultured at 37 degrees C were labeled with Cy3, and the cDNAs derived from those cultured at 16 degrees C were labeled with Cy5, followed by competitive hybridization on the same random genomic DNA array. Fig. 7B shows the analysis results from the low temperature stimulation, where the horizontal axis represents signal values derived from the culture at 16 degrees C and the vertical axis represents signal values derived from the culture at 37 degrees C in the plot.

In either analysis, a plurality of fixed DNA fragments that have values deviated from the two fold up- or down-regulation lines shown by the red broken lines in the graph were confirmed. Thus, it was verified that the difference in gene expression due to culture conditions can be detected by the random genomic DNA array constructed according to the present invention.

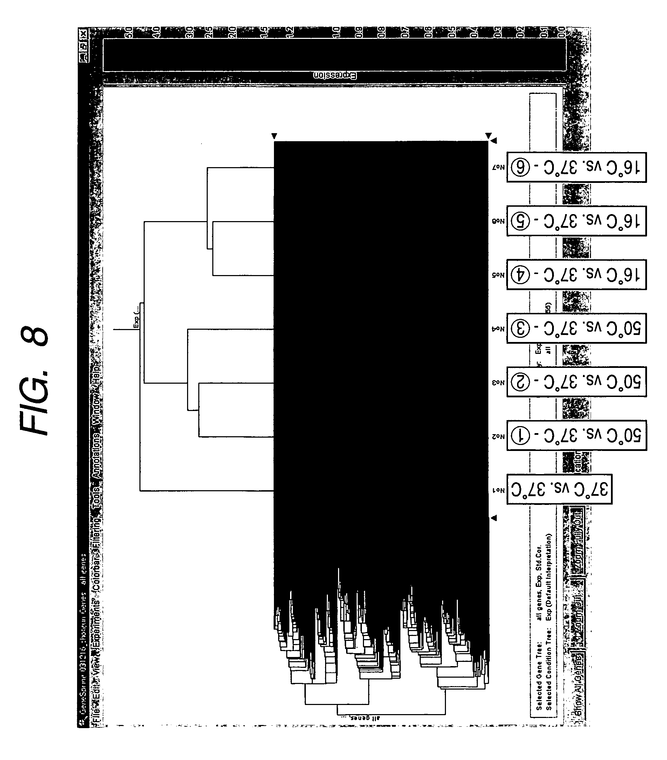

The above analyses of the up- or down-regulation caused by the temperature stimulation were repeated three times, respectively, and those results were subjected to cluster analysis, the results of which are shown in Fig. 8. The analysis by the present random genomic array showed high reproducibility as shown by the image data in Fig. 8. Furthermore, when clustering among individual arrays was carried out using the expression intensity of the total spot as an index, each condition of culture temperature was clustered as shown in the upper portion of Fig. 8. The results indicate that it is possible to judge easily the growth condition of the present organism by clustering the results of gene expression analysis when representative expression data under ideal growth conditions or the like has been already acquired. Assessment of culture condition by such expression analysis is considered to be extremely useful particularly for a production site of organic materials or food by fermentation technology.

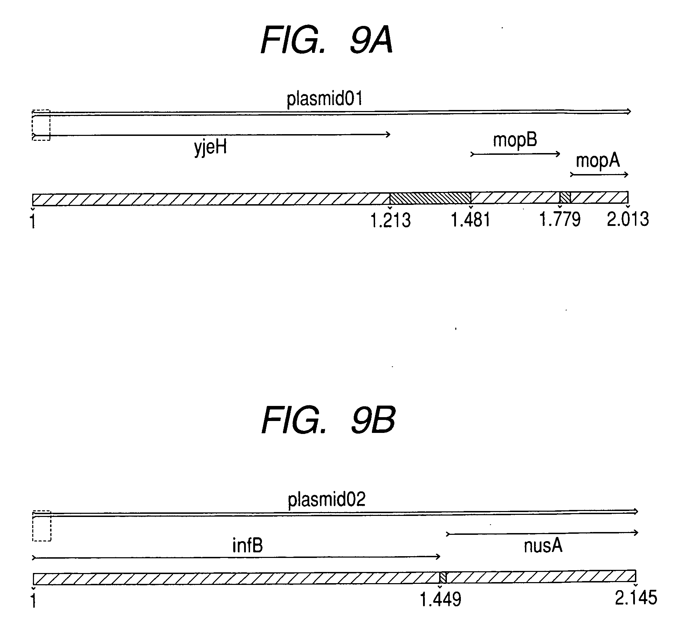

Next, the validity of the up- or down-regulation was verified. The clones up-regulated or down-regulated by the high temperature stimulation were analyzed for their sequences. The obtained sequences were searched for homology in the genome data of *E. coli* K-12 provided by NCBI (National Center for Biotechnology Information (http://www.ncbi.nlm.nih.gov/)). Representative examples of the results are shown in Fig. 9. Fig. 9A represents the result of homology search of the sequence analyzed for a plasmid (plasmid01) that showed an increased expression by the treatment at 50 degrees C. The presence of three kinds of genes (yjeH, mopB, mopA) was confirmed. It has been reported in Non-patent document 3 that the expression of all of these genes was increased in *E. coli* cultured at high temperature. Moreover, Fig. 9B represents the result of homology search of the sequence analyzed for a plasmid (plasmid02) that showed an increased expression by the treatment at 16 degrees C. The presence of two kinds of genes (infB, nusA) was confirmed. It has been reported in Non-patent document 4 that the expression of both of these genes was increased in *E. coli* cultured at low temperature.

These results indicate that the global monitoring of gene expression obtained by the use of the array constructed according to the present invention gives rise to reliable results.

### Example 2 Construction of sub DNA array

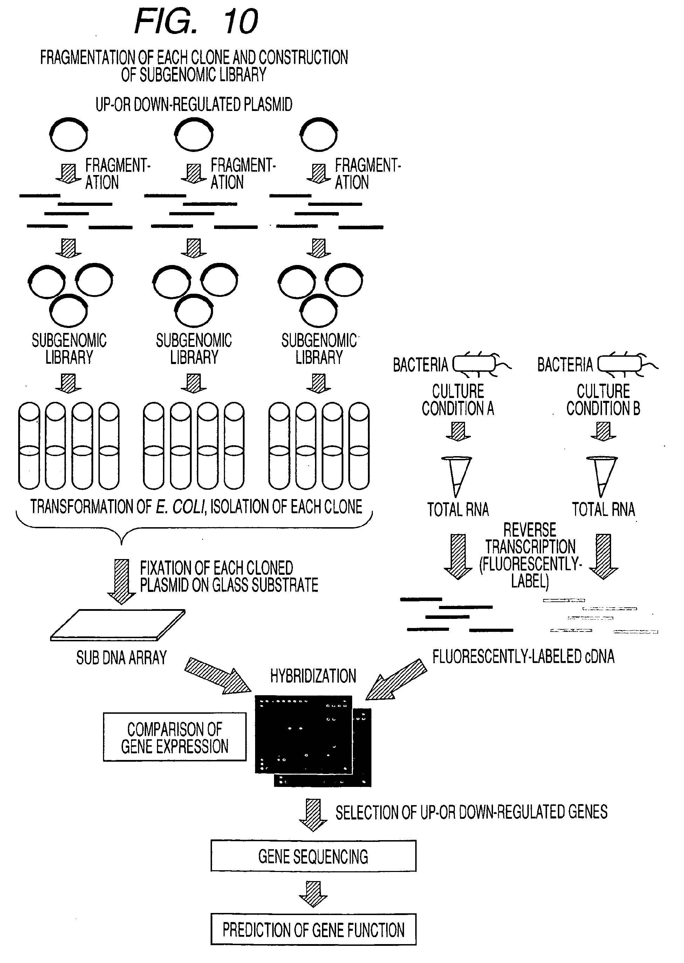

Fig. 10 is an outline scheme for construction of a sub DNA array. Individual up- or down-regulated plasmids detected by the random genomic DNA array are respectively digested into fragments of ca. 500 bp with a restriction enzyme or by physical shearing, and then a plasmid library is obtained by ligating into a plasmid vector. Although the number of colonies to be recovered varied depending on the length of genomic DNA fragments that had been inserted into the plasmids before the digestion, it is desirable to obtain a colony number resulting in redundancy of at least five in order to secure global monitoring.

The obtained plasmid libraries are spotted on a slide glass treated with poly-lysine using the spotter SP-Bio (manufactured by Hitachi Software Engineering Co., Ltd.) to obtain the sub DNA array by heat treatment at 80 degrees C.

Hybridization is carried out using labeled cDNAs, and their up- or down-regulations are analyzed for individual spots.

The clones up- or down-regulated are analyzed for their sequences, and gene prediction from the sequences and prediction of gene function by homology search using the database provided by NCBI etc. are carried out.

The genomic DNA fragments fixed on the present sub DNA array are approximately 500 bp. Therefore, these are considered to be short enough to contain only single genes, and it is possible to identify easily genes truly up- or down-regulated by means of sequencing.

In the present method, genes truly up- or down-regulated can be efficiently identified from the nucleic acid array fixed with genomic fragments that may include a plurality of genes. Since the fixed molecules may include a plurality of gene species in the random genomic DNA array of the present invention, global analysis of target organism condition can be performed without depending on identification of gene species. An analysis from such a viewpoint is useful when the reference strain and related strain of a microorganism, which differ in growth, states respectively depending on conditions are analyzed or when the reference strain and mutant strain of a microorganism are analyzed for their respective presence and behaviors are analyzed. Further, in the subgenomic DNA array of the present invention, it is expected to contain only single gene in each subgenomic DNA fragments owing to short fragmentation despite the fact that genomic DNA fragments are used as probes. This allows truly up- or down-regulated genes to be identified with ease when combined with sequencing. The combined use of the random genomic DNA array and the subgenomic DNA array makes it possible to identify genes up- or down-regulated, without preliminary sequencing, with respect to nucleic acids derived from an organism of which genomic DNA sequences have not been fully elucidated. In other word, global monitoring of gene expression followed by efficient gene analysis can be carried out even when the accumulation of past research is small, and is particularly useful for the study of genetic breeding of living organisms and so on.

### Example 3 Expression analysis of mutant bacteria and relative bacteria

When a mutant bacterium with a phenotype different from the reference bacterium is present, it is possible to carry out expression analysis of these bacteria, respectively, by using a shotgun genomic array constructed from a genomic DNA library of the reference bacterium or the mutant bacterium, or by using an array on which each genomic library from the respective bacteria is fixed on the same substrate. The difference in gene expression obtained by the present analysis is considered to be related to the difference in phenotypes between these bacteria.

For example, genes contributing to an increase of productivity or genes contributing to a decrease of productivity can be detected by comparing gene expression between a standard bacterium (α bacterium) and a mutant bacterium with high productivity (β bacterium) or a mutant bacterium with low productivity (γ bacterium). Those genes that modulate productivity can be used for breeding of organisms by genetic engineering and the like, and are very important in industrial application. It should be noted that analysis of relative bacteria is also possible in a similar manner.

### Example 4 Use of analysis results from random genomic array

The random genomic array is provided with a number of DNA fragments fixed on a substrate to achieve high global monitoring. However, once DNA fragments to which attention should be paid have been determined from the result of expression analysis, it seems unnecessary to continue global monitoring with the random genomic array.

Conceivable utilization methods of analysis results from the random genomic array include, for example, 1) to construct a random genomic array on which an arbitrary number of fixation probes selected from the original random genomic array are fixed, 2) to construct a DNA array on which oligonucleotides prepared based on sequencing of an arbitrary number of fixation probes selected from the original random genomic array are fixed, and 3) to analyze gene expression by PCR with the use of primers based on sequencing of an arbitrary number of fixation probes selected from the original random genomic array. The utilization of analysis results from the random genomic array is achieved more efficiently by analysis according to these methods 1) to 3).

[Non-patent document 3] Richmond et al., (1999) Nucleic Acid Research 27:3821-3835
[Non-patent document 4] Bae et al., (2000) Proc. Natl. Acad. Sci. USA 97:7784-7789

## Claims

1. A method of DNA array construction, comprising steps of:
isolating a genomic DNA from arbitrary cells;
preparing plasmids that contain genomic DNA fragments obtained by fragmentation of the genomic DNA; and
fixing the plasmids that contain the genomic DNA fragments on a substrate.

2. A method of DNA array construction, comprising steps of:
isolating a genomic DNA from arbitrary cells;
preparing plasmids that contain genomic DNA fragments obtained by fragmentation of the genomic DNA;
selecting arbitrary plasmids from the plasmids that contain the genomic DNA fragments with the use of a first substrate on which the plasmids that contain the genomic DNA fragments are fixed;
preparing plasmids that contain fragments of the arbitrary plasmids obtained by fragmentation of the arbitrary plasmids; and
fixing the plasmids that contain the fragments of the arbitrary plasmids on a second substrate.

3. A method of DNA analysis, comprising steps of:
preparing a first group of nucleic acid molecules that contain at least part of the sequence of a genomic DNA with the use of a genomic library constructed from at least part of the genomic DNA;
fixing the first group of nucleic acid molecules on a first substrate; and
supplying nucleic acids to be analyzed to the first substrate and analyzing the results from hybridization between the first group of nucleic acid molecules and the nucleic acids to be analyzed.

4. The method of DNA analysis according to claim 3, further comprising steps of:
selecting arbitrary nucleic acid molecules from the first group of nucleic acid molecules based on the results from the hybridization;
fragmenting the arbitrary nucleic acid molecules to construct a subgenomic library consisting of a group of short fragments;
preparing a second group of nucleic acid molecules that contain at least part of the sequences of the arbitrary nucleic acid molecules with the use of the subgenomic library;
fixing the second group of nucleic acid molecules on a second substrate; and
supplying the nucleic acids to be analyzed to the second substrate and analyzing the results from hybridization between the second group of nucleic acid molecules and the nucleic acids to be analyzed.

5. The method of DNA analysis according to claim 3, wherein the nucleic acids to be analyzed are cDNAs prepared using as templates mRNAs that are obtained from an organism having the genomic DNA as its genomic DNA.

6. The method of DNA analysis according to claim 4, wherein the step of selecting the sequences of arbitrary nucleic acid molecules includes homology search of the sequences of arbitrary nucleic acid molecules and analysis of genes contained in the arbitrary nucleic acid molecules.

7. The method of DNA analysis according to claim 4, wherein the length of the group of short fragments is shorter than the length of expected genes when the genomic DNA is derived from a prokaryote or the length of exons when the genomic DNA is derived from a eukaryote.

8. The method of DNA analysis according to claim 3, wherein the genomic DNA is derived from cells before changing culture conditions and the nucleic acids to be analyzed are derived from the cells after changing culture conditions; and gene expression before and after changing the culture conditions is analyzed in the step of analyzing the results from hybridization between the first group of nucleic acid molecules and the nucleic acids to be analyzed.

9. The method of DNA analysis according to claim 3, further comprising steps of:
selecting arbitrary nucleic acid molecules from the first group of nucleic acid molecules based on the analysis results from the hybridization to fix on a third substrate; and
supplying the nucleic acids to be analyzed to the third substrate and analyzing the results from hybridization between the arbitrary nucleic acid molecules and the nucleic acids to be analyzed.
